(19) Europäisches Patentamt
European Patent Office
Office européen des brevets

(11) Publication number: 0 061 240
A2

# (12) EUROPEAN PATENT APPLICATION

(21) Application number: 82300988.1

(22) Date of filing: 25.02.82

(51) Int. Cl.$^3$: C 08 F 4/40
A 61 K 6/00

(30) Priority: 20.03.81 US 245933

(43) Date of publication of application:
29.09.82 Bulletin 82/39

(84) Designated Contracting States:
CH DE FR GB IT LI SE

(71) Applicant: MINNESOTA MINING AND MANUFACTURING COMPANY
3M Center, P.O. Box 33427
St. Paul, MN 55133(US)

(72) Inventor: Bunker, James E.
2501 Hudson Road P.O. Box 33427
Saint Paul Minnesota 55133(US)

(74) Representative: Baillie, Iain Cameron et al,
c/o Ladas & Parry Isartorplatz 5
D-8000 München 2(DE)

## (54) Dentin and enamel adhesive.

(57) Polymerizable compositions for use in dentistry and methods for employing the same, in which salts of sulfurous acid act as an aid to polymerization of the compositions and enhance adhesion of the compositions to dentin.

EP 0 061 240 A2

# DENTIN AND ENAMEL ADHESIVE

## Technical Field

This invention relates to the field of polymerizable compositions. In addition, this invention relates to compositions for use as liners, restoratives, and composites for the repair of teeth, and to compositions for use in fastening orthodontic brackets or crowns to teeth. This invention also relates to a method for repairing, adhering, or altering the position of teeth, through the use of such compositions as liners, restoratives, composites, and adhesives.

## Background Art

Practitioners in the field of dentistry have long sought polymerizable compositions which would adhere well to dentin. One of the first attempts at bonding to dentin was recorded by Buonocore et al, utilizing a polymerizable mixture containing glycerophosphate dimethacrylate (I), together with *p*-toluenesulfinic acid (II) as catalyst:

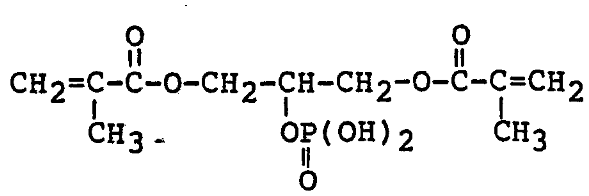

I

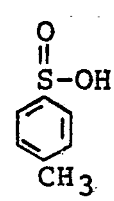

II ,

see M. Buonocore, W. Wileman, and F. Brudevold, J. Dent. Res., 35, 846 (1956), and M. Buonocore and M. Quigley, J. Amer. Dent. Assoc., 57, 807 (1958). The resulting resin was said to have excellent adhesion to dentin, but a tendency toward brittleness and gellation.

Compositions containing a salt of a sulfinic acid (e.g., sodium benzene sulfinate) or a salt of a

sulfonic acid (e.g., sodium benzene sulfonate) and various phosphoric acid or phosphonic acid esters have been described as having good adhesion to dentin in patent applications and patents, see, e.g., U. S. Patent nos. 4,182,035, 4,222,780, and 4,235,633, O.L.S. Nos. 2711234 and 2818068, and Japanese laid-open application nos. 77-113089, 78-30193, 78-39331, 78-67740, 78-69494, 78-110637, 78-113843, 78-134037, 78-144939, 78-138441, 79-21438, and 79-28339. Also, there has been introduced in Japan a dental liner composition, under the name "Clearfil Bond System F" (hereinafter, "Clearfil"), utilizing a two-part resin system. The first (catalyst) portion of such resin system contains a polymerizable phosphoric acid of undetermined structure. The second (universal) part of such resin system contains an ethanolic solution containing 3 weight percent sodium benzene sulfinate and 1 weight percent N,N-dihydroxyethyl-p-toluidine (the latter compound will be referred to hereafter as "DHPT"). It has been recommended that the use of this composition be preceeded by acid etching of the exposed dentin (e.g., with ortho-phosphoric acid) prior to application of the liner composition. However, the long term physiological affects of such acid etching are unknown, and the efficacy of acid etching of dentin has been questioned by practitioners, see, e.g., M.G. Buonocore, "The Challenge of Bonding to Dentin", The Acid Etch Technique, L. M. Silverstone and I. L. Dogon, Eds., Proceedings of an International Symposium at St. Moritz, Switzerland, Dec. 16-18, 1974, North Central Publishing Co. (St. Paul, 1975). Also, acid etching is a somewhat difficult procedure to carry out, since the highly corrosive acid is injurious to the soft tissues of the mouth. In addition, commercial products containing ortho-phosphoric acid are, in some jurisdictions, subjected to special transportation requirements which increase the costs of shipping dental supplies (e.g. restoration kits) which contain vials of ortho-phosphoric acid.

A dentin adhesive composition should desirably offer good adhesion to both dentin and tooth enamel, as well as adhering well to other restorative and composite resins, crowns, and/or orthodontic brackets currently in use ("restorative" and "composite" will be used essentially interchangeably herein, in recognition of the fact that due to differing standards currently in effect throughout the world, an individual dental adhesive composition might be regarded as a "restorative" in some jurisdictions and as a "composite" in others). Also, a dentin adhesive composition should desirably reduce the need for detailed cavity preparation such as undercutting. In addition, a dentin adhesive composition should withstand repeated thermally-induced expansion and contraction while minimizing marginal leakage between the adhesive composition and adjacent tooth tissue or restorative or composite materials. Also, it would be desirable if a dentin adhesive composition offered sufficiently strong bonding to dentin and enamel that the acid etching technique currently used for most dental restorations could be eliminated.

## Disclosure of Invention

The present invention provides, in one aspect, polymerizable compositions having particularly valuable use in dentistry, comprising a free-radically polymerizable liquid monomer and a sulfur compound, characterized in that said sulfur compound is a salt of sulfurous acid (e.g., a bisulfite, metabisulfite, hydrosulfite, or sulfite salt of the acid $H_2SO_3$).

In addition, the present invention provides dental liner, restorative, composite, or adhesive compositions comprising said monomer and said salt of sulfurous acid, and optionally further comprising at least one tertiary amine, and at least one free-radical initiator, the resulting compositions being packaged in a stable, conveniently mixable configuration. Also, the present invention provides a method for using said liner,

restorative, and composite compositions to repair or veneer hard dental tissue, and a method for applying orthodontic brackets or crowns to hard dental tissue using said adhesive compositions.

## Detailed Description

In the practice of the present invention, the free-radically polymerizable liquid monomer is a monomer which can be cured or hardened to a solid, higher molecular weight state using a source of free radicals. Suitable free-radically polymerizable liquid monomers having utility in dental applications include diglycidyl methacrylate of Bisphenol A (hereafter referred to as "BIS-GMA"), glycerophosphate dimethacrylate (hereafter referred to as "GPDM"), organic esters of one or more acids of phosphorus, said esters having chlorine or bromine bonded directly to phosphorus, and the organic radical of said esters containing at least one polymerizable functional group (hereafter referred to as "preferred phosphorus acid esters"), and other phosphorus acid esters such as O-phenyl-O-methacryloxyethyl phosphoric acid.

BIS-GMA, GPDM, O-phenyl-O-methacryloxyethyl phosphoric acid, and other similar polymerizable liquid monomers are well-known to those skilled in the art. Said preferred phosphorus acid esters can be synthesized as described in more detail below.

The preferred phosphorus acid esters can be characterized by the formulas (III) and (IV):

$$(R^1-O)_m\underset{(\overset{\|}{O})_p}{-P-}(X)_n$$

III

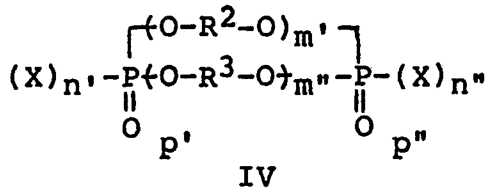

IV

wherein

m is 1 to 3,

m' and m" are zero or 1 and are the same or different,

n is 1 to 4,

n' and n" are independently zero to 4 and are the same or different, with the proviso that n' and n" are both not zero,

p, p', and p" are zero or 1 and are the same or different,

m+n+2p = 3 or 5,

m'+m"+n'+2p' = 3 or 5,

m'+m"+n"+2p" = 3 or 5,

$R^1$ is a monovalent olefinic organic radical (preferably alkenyl, alkenoxy, cycloalkenyl, aralkenyl, or alkenaryl, having 2 to 40 carbon atoms) which can be straight chain, branched, or cyclic, can contain skeletal hetero atoms, i.e., atoms other than carbon (e.g., oxygen, sulfur, or non-basic nitrogen atoms), and can be unsubstituted or substituted with non-interfering moieties, e.g., moieties which do not interfere with free-radical polymerization of said phosphorus acid esters,

$R^2$ and $R^3$ are divalent olefinic organic radicals (preferably alkenylidene, oxyalkenylidene, cycloalkenylidene, arylenealkenylidene, or alkenylidenearylene, having 2 to 40 carbon atoms) which can be straight chain, branched, or cyclic, can contain skeletal hetero atoms, can be unsubstituted or substituted with non-interfering moieties, and are the same or different, and

X is Cl, Br, or $R^4$, where $R^4$ is an aliphatic or oxyaliphatic radical having 1 to 12 carbon atoms, and each X is the same as or different from other X, with the proviso that at least one X is Cl or Br.

Compounds of formula III and IV contain trivalent or pentavalent phosphorus atoms. In compounds of formula III, phosphorus is bonded to at least one chlorine or bromine atom. In compounds of formula IV, at least one phosphorus atom is bonded to at least one chlorine or bromine atom. Preferably phosphorus is bonded to chlorine. The preferred phosphorus acid esters desirably contain at least one double bond between phosphorus and oxygen or sulfur, with a double bond to oxygen being preferred. Most preferably two or more polymerizable functional groups per phosphorus atom are contained in the preferred phosphorus acid esters. Also, the preferred phosphorus acid esters are preferably liquids at room temperature.

The polymerizable functional group in the preferred phosphorus acid esters is a free-radically polymerizable group, such as an olefin, and is most preferably a monofunctional or difunctional acryl or methacryl radical. Other polymerizable functional groups include monofunctional or difunctional vinyl, allyl, crotyl, and cinnamyl radicals.

Representative compounds of formula III include:

$$CH_2{=}C(CH_3)C(O)OC_2H_4O\overset{\displaystyle Cl}{\underset{\displaystyle Cl}{\overset{|}{\underset{|}{P}}}}{=}O$$ ,

$$CH_2{=}C(H)C(O)OC_2H_4O\overset{\displaystyle Ø}{\underset{\displaystyle Cl}{\overset{|}{\underset{|}{P}}}}{=}O$$ (where "Ø" is a phenyl radical),

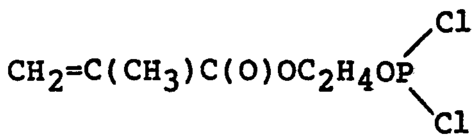,

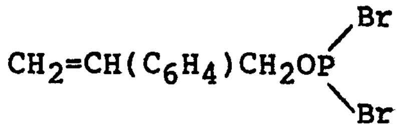,

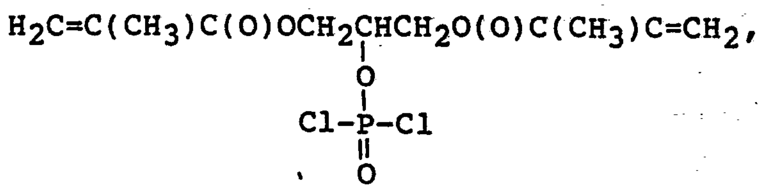,

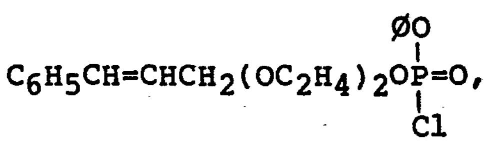 and

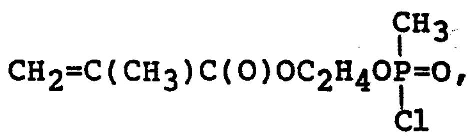,

as well as mixtures of more than one of the above compounds.

Representative compounds of formula IV include:

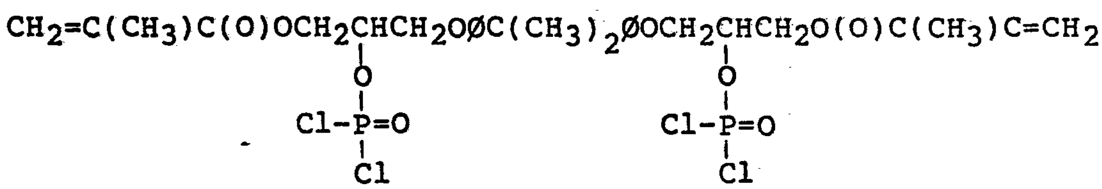,

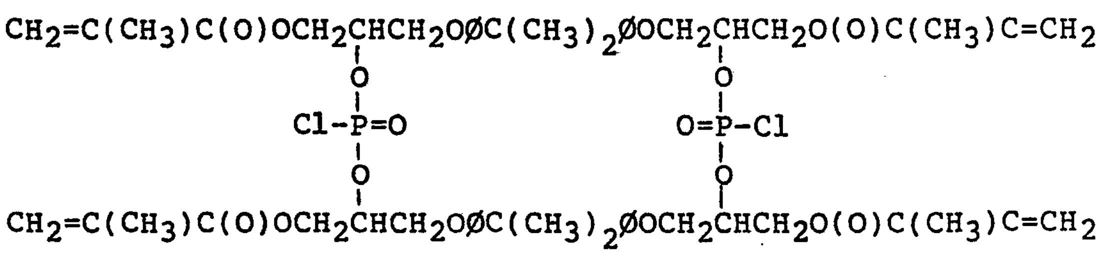,

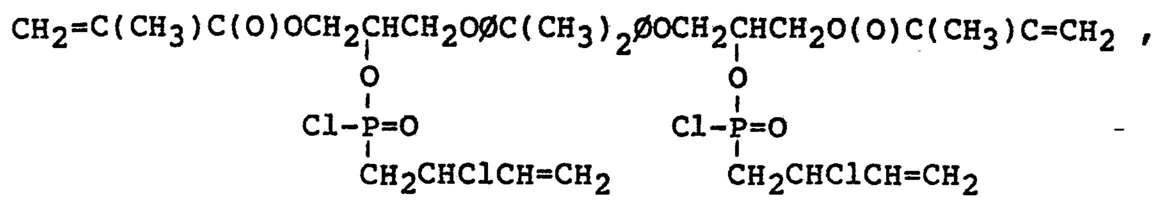

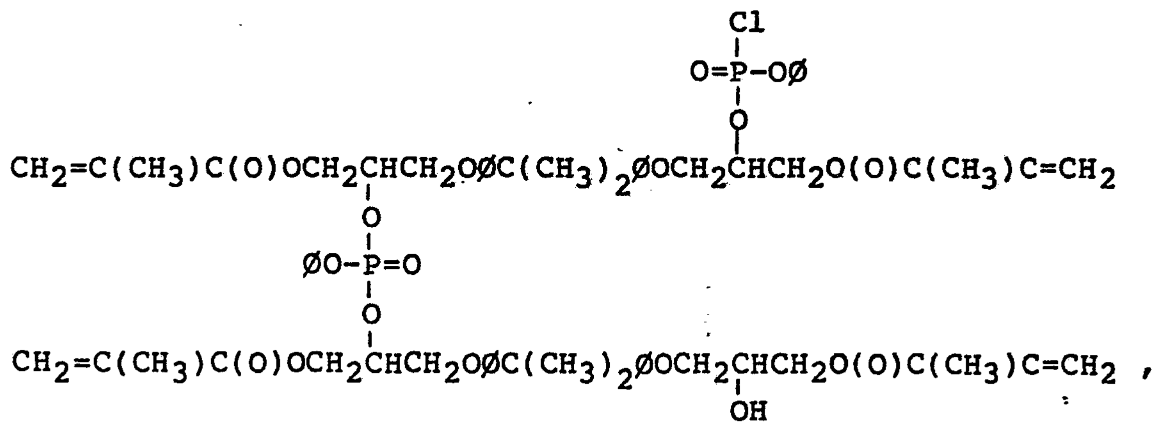

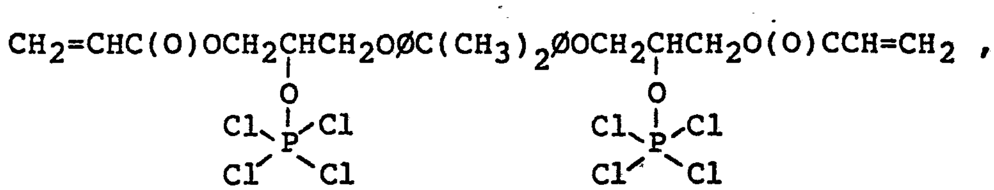

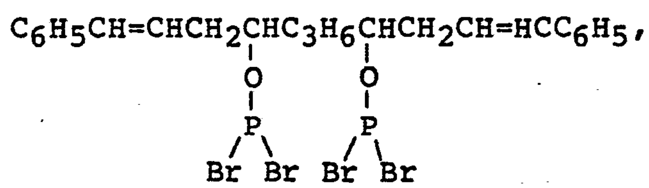

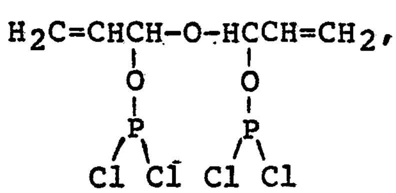

and

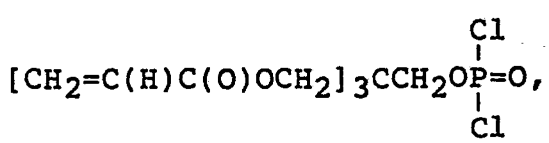

as well as mixtures of more than one of the above compounds.

The preferred phosphorus acid esters can be used individually or in the form of adducts containing more than one phosphorus acid ester. Preferably, the preferred phosphorus acid esters are prepared by combining a chlorine- or bromine-containing phosphorus acid (e.g., phosphorus oxychloride, $POCl_3$, also known as phosphoryl chloride) with a polymerizable monomer having at least one reactive hydroxyl group (e.g., BIS-GMA). Such polymerizable monomers having at least one reactive hydroxyl group will be hereafter referred to as "hydroxylated monomers". When the hydroxylated monomer has a high initial viscosity, it is preferable to mix the phosphorus acid with the hydroxylated monomer and a suitable diluent, e.g., triethyleneglycol dimethacrylate.

The phosphorus acid and hydroxylated monomer will react at low temperature, e.g., at room temperature, and the reaction mixture will increase in viscosity, preferably reaching an equilibrium state that is stable over time. The reaction product of such a mixture will generally be an adduct, the phosphorus acid esters of which are the product of reactions between some or all of the various hydroxyl groups of the hydroxylated monomer and available chlorine or bromine atoms of the phosphorus acid. Sufficient phosphorus acid should be added to the hydroxylated monomer to provide good bonding and handling performance in liner, restorative, or composite compositions prepared therewith. For an adduct prepared by combining phosphorus oxychloride and BIS-GMA, about 0.25 to twenty percent by weight phosphorus oxychloride, and preferably about one to ten percent by weight phosphorus oxychloride should be used, based on the weight of BIS-GMA. Because BIS-GMA contains two hydroxyl groups per molecule, the above weight percentage values represent equivalent ratios of $POCl_3$ to BIS-GMA of about 0.025:1 to 1:1, preferably about 0.05:1 to 0.5:1. Suitable adjustment of such equivalent ratios should be made when the preferred phosphorus acid esters are prepared from

hydroxylated monomers having other hydroxyl functionality, e.g., monofunctionality or trifunctionality. Also, suitable adjustment of such equivalent ratios should be made when the preferred phosphorus acid esters are prepared from phosphorus acids other than phosphorus oxychloride. Expressed in terms of the ratio of halogen atoms in the phosphorus acid to hydroxyl groups in the hydroxylated monomer, the phosphorus acid and hydroxylated monomer should be combined in a ratio of halogen atom to hydroxyl group between about 0.0375:1 to 1.5:1, preferably about 0.075:1 to 0.75:1.

If lesser amounts of phosphorus acid than those amounts sufficient to provide good bonding and handling performance are used, the resulting adduct may have low adhesion to dentin and enamel when polymerized therewith. If larger amounts of phosphorus acid than those sufficient to provide good bonding and handling are used, the resulting adduct will tend to homopolymerize, thereby having inadequate shelf life.

Other phosphorus acids which can be reacted with hydroxylated monomers include $CH_3POCl_2$, $PCl_3$, $PCl_5$, $C_6H_5POCl_2$, $C_6H_5OPOCl_2$, and $PBr_3$. Such phosphorus acids can be used singly or in combination. Phosphorus oxychloride is a preferred phosphorus acid for use in the preparation of the preferred phosphorus acid esters used in this invention.

Other hydroxylated monomers which can be used in this invention include hydroxyethyl methacrylate, pentaerythritol triacrylate, glycerol dimethacrylate, methyl vinyl alcohol, vinyl benzyl alcohol, allyl alcohol, crotyl alcohol, and cinnamyl alcohol.

The mixing of phosphorus acid and hydroxylated monomer can be carried out at room temperature. The attainment of equilibrium between the phosphorus acid and hydroxylated monomer can be determined by observing the viscosity of the adduct over time, with equilibrium being indicated by a leveling off of such viscosity.

The salts of sulfurous acid which are used in this invention can be characterized by the formulas V, VI, VII and VIII:

$MHSO_3$ ,
V

$M_2S_2O_5$ ,
VI

$M_2S_2O_4$ , and
VII

$M_2SO_3$
VIII

wherein M is a metal from Group I of the Periodic Table of the Elements, or a cation of the formula $N(R^5)_4^+$ where $R^5$ is a hydrogen atom, a monovalent alkyl or cycloalkyl radical having about 1 to 8 carbon atoms, or two $R^5$ taken together with the nitrogen atom to which they are joined combine to form a 5 to 7 membered ring, each $R^5$ is the same as or different from other $R^5$, and $R^5$ can contain hetero atoms which do not interfere with the functioning of the salt of sulfurous acid as an aid to polymerization of the polymerizable liquid monomer, such as oxygen, sulfur, or nitrogen. Such salts are ordinarily alkali metal salts, such as potassium or sodium salts or ammonium or alkylammonium salts, of bisulfite, metabisulfite, hydrosulfite, or sulfite anions.

Suitable compounds of formulas V, VI, VII, and VIII include $LiHSO_3$, $NaHSO_3$, $KHSO_3$, $NH_4HSO_3$, $Li_2S_2O_5$, $K_2S_2O_5$, $Na_2S_2O_5$, $(NH_4)_2S_2O_5$, $Na_2S_2O_4$, $K_2S_2O_4$, $(NH_4)_2S_2O_4$, $Li_2SO_3$, $Na_2SO_3$, $K_2SO_3$, $(NH_4)_2SO_3$, and mixtures thereof. Bisulfites, and especially ammonium bisulfite, are preferred. Generally, the liner, restorative, composite and adhesive compositions of this invention contain about 0.5 to 10 percent by weight of salt of sulfurous acid, and

preferably contain about 3 to 6 percent by weight of salt of sulfurous acid.

The salts of sulfurous acid used in this invention accelerate the rate of polymerization of polymerizable monomers customarily used in dentistry (e.g., BIS-GMA). For example, a freshly-prepared mixture of an alcoholic solution containing 4.5 weight % ammonium bisulfite and 2 weight % DHPT in 50% aqueous ethanol with an equal volume of a polymerizable monomer mixture containing 49 weight % BIS-GMA, 1 weight % benzoyl peroxide, 49 weight % triethyleneglycol dimethacrylate, 0.1 weight % butylated hydroxytoluene, 0.2 weight % phenyl solicylate, and 0.1 weight % glycidyl methacrylate yielded a liquid composition which could be spread into a thin film and hardened within 20 seconds after the start of mixing. In contrast, when ammonium bisulfite was omitted from the above-described liquid composition, hardening of a thin film took 100 seconds. When sodium *p*-toluenesulfinate was substituted for ammonium bisulfite, hardening took 200 seconds.

In addition, the salts of sulfurous acids used in this invention provide liner, restorative, composite, and adhesive compositions having very good adhesion to etched and unetched dentin and etched and unetched enamel. Preferably the compositions of this invention are used in the form of liners and are applied to unetched dentin.

The liner, restorative, composite, and adhesive compositions of the invention optionally contain a tertiary amine which acts as a polymerization accelerator, in amounts of about 0.5 to 10 percent by weight. Suitable tertiary amines include DHPT, N,N-dimethyl-para-toluidine, N,N-bis(2-hydroxyethyl)-3,5-xylidine, and the like. DHPT is a preferred tertiary amine. The amount of accelerator used in compositions of this invention can be reduced if ammonium bisulfite is used as the salt of sulfurous acid in such compositions.

Liner, restorative, composite, and adhesive compositions of this invention typically contain a polymerization catalyst in amounts of about 0.05 to 5 percent by weight. Suitable polymerization catalysts include free-radical initiators such as peroxides, e.g., benzoyl peroxide, acetyl peroxide, lauroyl peroxide, and t-butyl hydroperoxide. Benzoyl peroxide is a preferred catalyst. Photoinitiators (i.e., light-activatable catalysts) such as monoketals of aromatic 1,2-diketones or a combination of benzil and a dialkylamino acrylate or methacrylate can also be used.

When the compositions of the invention are used as liners which are then covered with polymerizable restorative or composite compositions, the liner composition need not contain accelerator or catalyst so long as sufficient accelerator or catalyst can migrate from the polymerizable restorative or composite composition into the liner composition, thereby promoting polymerization of the resin in the liner. However, for optimum reproducibility in use, liner compositions of this invention typically contain measured amounts of salt of sulfurous acid, accelerator, and catalyst.

Other adjuvants such as solvents, stabilizers, fillers, pigments, inhibitors, and the like can also be used in the compositions of this invention. The amounts and types of such adjuvants, and their manner of addition to the compositions of this invention will be essentially the same as currently used in existing liner, restorative, composite, or adhesive compositions familiar to those skilled in the art. Ethanol and aqueous ethanol are preferred solvents for use in liner compositions of this invention. Quartz, and glasses such as zinc glass or other radiopaque glass treated with appropriate silane surface treatment, are preferred fillers for use in restorative and composite compositions of this invention. Asbestos-free talc is a preferred filler for use in adhesive compositions of this invention.

The liner compositions of the invention are preferably put up in multiple-part packages. For example, a suitable solvent (e.g., aqueous ethanol), salt of sulfurous acid, and accelerator can be combined in a first part, and free-radically polymerizable liquid monomer and catalyst can be combined in a second part. While uncombined, the resulting two-part package will remain in a stable, uncured state. When the two parts are mixed together, e.g., by spatulation or other means, the resulting liner composition will rapidly cure. Also, a suitable first solvent (e.g., aqueous ethanol or water) and salt of sulfurous acid can be combined in a first part, a suitable second solvent (e.g., aqueous or absolute ethanol) and accelerator can be combined in a second part, and free-radically polymerizable liquid monomer and catalyst can be combined in a third part, and the three parts later mixed together for use. The amount of each ingredient in such multiple-part packages should be adjusted to allow sufficient working time for the practitioner to mix and apply the liner composition as desired, together with attainment of the desired physical properties in the cured liner.

If desired, other combinations of free-radically polymerizable liquid monomer, salt of sulfurous acid, accelerator, catalyst, and any other desired adjuvants can also be employed in multiple-part packages of liner compositions of this invention. Preferably, a multiple-part liner composition package offers ease of mixing, good shelf life, and desirable physical properties after cure.

Multiple-part packages of restorative, composite, and adhesive compositions of this invention are preferred and can be prepared, for example, by combining free-radically polymerizable liquid monomer, catalyst, and filler in a first part, and salt of sulfurous acid, accelerator, and a suitable solvent (preferably aqueous ethanol) in a second part. While uncombined, the resulting two-part package will remain in a stable, uncured state. When the two parts are mixed together, e.g., by spatulation or other

means, the resulting restorative, composite, or adhesive composition will rapidly cure. The amount of each ingredient in such two-part package should be adjusted to allow sufficient working time and attainment of desired physical properties.

If desired, other combinations of free-radically polymerizable liquid monomer, salt of sulfurous acid, accelerator, catalyst, filler, and any other desired adjuvants can also be employed in multiple-part packages of restorative, composite, and adhesive compositions of this invention, coincident with attainment of ease of mixing, good shelf life, and desirable physical properties after cure.

When used as liners, the compositions of this invention are applied in a manner similar to that used for existing dental liner compositions. However, cavity preparation is simplified. Excavation can be limited to the removal of damaged or defective tooth structure. Undercutting of the cavity is generally not required. If desired, acid etching of the cavity can be omitted. This invention therefore shortens the time required for completion of a dental restoration and reduces trauma to healthy tooth structure.

When used as a composite or restorative, the compositions of this invention are used in a fashion similar to that used for existing dental composites and restoratives. Preferably, where the compositions of this invention are used as composites or restoratives, they are used in conjunction with a liner prepared according to this invention which is applied to the excavated cavity prior to application of the composite or restorative composition.

When used as an orthodontic bracket adhesive, the compositions of this invention are preferably used as primers in conjunction with existing filled orthodontic bracket adhesives. The compositions of the invention can also be combined with fillers and used in place of such

adhesives, preferably in conjunction with a primer (i.e., liner) prepared according to the present invention. Where desired, e.g., to obtain very high bonding strength, acid etching of the exposed tooth enamel can be employed. However, satisfactory results can often be obtained in the absence of such acid etching, thereby reducing damage to enamel.

The following examples are offered to aid understanding of the present invention and are not to be construed as limiting the scope thereof.

EXAMPLE 1

Preparation of a Dental Liner Composition

5 g of phosphorus oxychloride was dissolved in a polymerizable monomer mixture (identified hereafter as "Resin A") containing 96 g of BIS-GMA, 2.0 g of benzoyl peroxide, 96 g of triethyleneglycol dimethacrylate, 0.13 g of butylated hydroxytoluene, 0.34 g of phenyl salicylate, and 0.24 g of glycidyl methacrylate. The resulting mixture was allowed to stand at room temperature for 5 days.

The above reaction product was used as the first part of a two-part liner composition. The second part of such liner composition was a solution of three percent by weight ammonium bisulfite and one percent by weight DHPT in 50 percent aqueous ethanol. Adhesion of this liner composition to unetched dentin was evaluated using the following procedure. Four bovine teeth of similar age and appearance were partially embedded in circular acrylic disks. The exposed portion of each tooth was ground flat and parallel to the acrylic disk using 120 grit silicon carbide paper-backed abrasive mounted on a lapidary wheel, in order to expose the tooth dentin. During this and subsequent grinding and polishing steps, the teeth were continuously rinsed with water. Further grinding and polishing of the teeth was carried out by mounting 400

grit silicon carbide paper-backed abrasive, and 600 grit alumina rubber-backed abrasive on the lapidary wheel.

The teeth were then washed with distilled water using a "Water Pik" apparatus set on "hard" for 15 seconds, followed by drying with air. One drop of each part of the above two-part liner composition was placed on a mixing pad. The drops were mixed together by hand spatulation for about 5 seconds, painted onto the polished tooth surface, and blown into a thin film with compressed air. A previously prepared "Teflon" mold having a 5 mm diameter hole lined with a gelatin sleeve was clamped around the tooth so that the central axis of the hole in the mold was normal to the polished, liner-coated tooth surface. The cavity in the mold was filled with a standard, premixed dental composite ("Concise" brand, commercially available from 3M). The tooth and mold were allowed to stand for about 10 minutes at room temperature. The mold was then carefully removed from the tooth, leaving a button-like molded composite shape attached to the liner layer. The disk-tooth-liner-composite combination was stored in distilled water at 37°C. for 24 hours.

Adhesion of the liner composition to the polished, unetched bovine dentin was evaluated by placing the tooth mounting disk in a holder and clamping the holder in the jaws of an "Instron" apparatus with the liner layer parallel to the direction of pull. A loop of orthodontic wire (0.44 mm diameter) was placed around the composite button adjacent to the polished tooth surface. The ends of the orthodontic wire were clamped in the pulling jaws of the Instron apparatus, thereby placing the liner bond in shear stress. At a crosshead speed of 5 mm/min, the average measured shear strength of the liner-dentin bond was 51 $kg/cm^2$.

In a comparison run, a two-part liner composition containing sodium benzene sulfinate in place of ammonium bisulfite was similarly evaluated. The

initial average bond strength of this composition was 53 kg/cm$^2$ on unetched dentin.

In an additional comparison run, "Clearfil" liner (commercially available from the Kuraray Co., Ltd.), was similarily evaluated. The initial average bond strength of "Clearfil" liner was 26 kg/cm$^2$ on unetched dentin. When "Clearfil" universal was combined with the above-described reaction product of $POCl_3$ and BIS-GMA, the initial average bond strength of the resulting composition was 54 kg/cm$^2$ on unetched dentin.

This example shows that the use of ammonium bisulfate in a dental liner composition provided very good adhesion results .

EXAMPLES 2-6

Liner compositions were prepared and evaluated according to the method of Example 1, but using other salts of sulfurous acids in place of ammonium bisulfite, and, in some examples, using larger amounts of $POCl_3$ in place of the 2.5 wt % $POCl_3$ in Resin A used in Example 1. In some cases, three-part systems were mixed together. Set out below in Table I are the Example No., composition (in weight percent) of each liner part used, and the bond strength of the resulting liner composition on unetched, polished dentin.

TABLE I

| Example no. | Part 1 | Part 2 | Part 3 | Bond strength, $kg/cm^2$ |
|---|---|---|---|---|
| 2 | 2.5% $POCl_3$ in Resin A | 5% $NaHSO_3$ in 40% aq. ethanol | 1.5% DHPT in abs. ethanol | 50 |
| 3 | 2.5% $POCl_3$ in Resin A | 4.4% $K_2S_2O_5$ in 33% aq. ethanol | 1.5% DHPT in abs. ethanol | 48 |
| 4 | 2.5% $POCl_3$ in Resin A | 4% $Na_2S_2O_4$ in 35% aq. ethanol | 1.5% DHPT in abs. ethanol | 40 |
| 5 | 5% $POCl_3$ in Resin A | 5% $NaHSO_3$ in 40% aq. ethanol | 1.5% DHPT in abs. ethanol | 31 |
| 6 | 5% $POCl_3$ in Resin A | 3% $NH_4HSO_3$ and 1% DHPT in 50% aq. ethanol | ——— | 30 |

EXAMPLES 7-12

Glycerophosphate dimethacrylate ("GPDM") was prepared by reacting glycerol and methacryloyl chloride at ice bath temperature, followed by addition of the resulting reaction product to a cold mixture of phosphorus oxychloride and triethylamine. Workup of the resulting reaction mixture was carried out without isolation of the intermediate. Hydrolysis, extraction and column chromatography on silica gel yielded the desired product as a yellow oil (50 percent yield, 92% purity, remainder glycerol).

Using the method of Example 1, but without polishing the teeth, liner compositions containing GPDM in Resin A, various salts of sulfurous acids, DHPT (in some examples), and suitable solvents were prepared and evaluated. Set out below in Table II are the example no., composition (in weight %) of each liner part used, and the

bond strength of the resulting liner composition on unetched, unpolished dentin.

The triethylammonium salts of Example Nos. 11 and 12 were prepared by titration of sulfurous acid with triethylamine to the first and second pH inflection points, respectively.

TABLE II

| Example no. | Part 1 | Part 2 | Part 3 | Bond strength, $kg/cm^2$ |
|---|---|---|---|---|
| 7 | 5% GPDM in Resin A | 3% $NH_4HSO_3$ and 1% DHPT in 50% aq. ethanol | — | 35 |
| 8 | 5% GPDM in Resin A | 5% $NaHSO_3$ in 40% aq. ethanol | 1.5% DHPT in abs. ethanol | 32 |
| 9 | 5% GPDM in Resin A | 6% $NaHSO_3$ in 40% aq. ethanol | — | 20 |
| 10 | 5% GPDM in Resin A | 4.5% $NH_4HSO_3$ in 50% aq. ethanol | — | 32 |
| 11 | 5% GPDM in Resin A | 6% $((C_2H_5)_3NH)HSO_3$ and 1.5% DHPT in abs. ethanol | — | 45 |
| 12 | 5% GPDM in Resin A | 6% $((C_2H_5)_3NH)_2SO_3$ and 1.5% DHPT in abs. ethanol | — | 17 |

EXAMPLE 13

Thermal Cycling

A three part liner composition was prepared using the method of Example 1. The first part contained 5% by weight GPDM in Resin A. The second part contained 6% by weight $NaHSO_3$ in 40% aqueous ethanol. The third part contained 1.5% DHPT in absolute ethanol. The three parts

were mixed and applied to polished, unetched dentin using the method of Example 1. The dentin-liner bonds were thermally cycled 330 times between 16°C and 50°C. After thermal cycling, the average measured shear strength of the liner dentin bond was 30 $kg/cm^2$.

In a comparison run, "Clearfil" liner was similarly evaluated. After thermal cycling, the average measured shear strength of the liner-dentin bond was 13 $kg/cm^2$.

This example shows that the bond strength of liner compositions of this invention is maintained even after exposure to cyclic thermal stress.

EXAMPLE 14

Primer for Orthodontic Bracket Adhesive

The liner composition of Example 6 was used as a primer for an orthodontic bracket adhesive. The liner composition was mixed and applied to tooth enamel which had been previously polished with 600 grit silicon carbide paper-backed abrasive, washed with a "Water Pik", and air-dried. A layer of standard orthodontic bracket adhesive ("Concise 1960" brand, commercially available from 3M) was applied to the back of an orthodontic bracket and pad (Bracket No. 007 and Pad No. 065, commercially available from American Orthodontics, Inc.). The adhesive-coated pad was applied to the primer and the resulting assembly allowed to cure for ten minutes at room temperature. The cured assemblies were stored in water at 37°C for 24 hours and then evaluated for average shear strength using the "Instron" apparatus described in Example 1. The average shear strength of the tooth-bracket bond was 69 $kg/cm^2$.

In a comparison run, sodium benzene sulfinate was substituted for $NH_4HSO_3$. The average shear strength of the tooth-bracket bond was 88 $kg/cm^2$.

This example shows that ammonium bisulfite could be effectively used in a primer for orthodontic bracket adhesives.

Various modifications and alterations of this invention will become apparent to those skilled in the art without departing from the scope and spirit of this invention, and it should be understood that this invention is not limited to the illustrative embodiments set forth herein.

CLAIMS:

1. Polymerizable compositions, comprising a free-radically polymerizable liquid monomer and a sulfur compound, characterized in that said sulfur compound is a salt of sulfurous acid.

2. Compositions according to Claim 1, further characterized in that said salt is a bisulfite, metabisulfite, hydrosulfite, or sulfite salt.

3. Compositions according to Claim 1, further characterized in that said salt has the formula $MHSO_3$, $M_2S_2O_5$, $M_2S_2O_4$, or $M_2SO_3$, wherein M is a metal from Group I of the Periodic Table of the Elements, or a cation of the formula $N(R^5)_4^+$ where $R^5$ is a hydrogen atom, a monovalent alkyl or cycloalkyl radical having 1 to 8 carbon atoms, or two $R^5$ taken together with the nitrogen atom to which they are joined combine to form a 5 to 7 membered ring, each $R^5$ is the same or different from other $R^5$, and $R^5$ can contain hetero atoms which do not interfere with the functioning of said salt of sulfurous acid as an aid to polymerization of said polymerizable liquid monomer.

4. Compositions according to Claim 3, further characterized in that M is a sodium, potassium, ammonium, or alkylammonium cation.

5. Compositions according to any preceeding claim, further characterized in that said salt of sulfurous acid is 0.5 to 10 percent by weight of the total weight of said composition.

6. Compositions according to any preceeding claim, further characterized in that said salt of sulfurous acid is 3 to 6 percent by weight of the total weight of said composition.

7. Compositions according to any preceeding claim, further characterized in that said compositions contain 0.05 to 5 percent by weight polymerization catalyst.

8. Compositions according to any preceeding claim, further characterized in that said polymerizable liquid monomer is selected from BIS-GMA, glycerophosphate dimethacrylate, O-phenyl-O-methacryloxyethyl phosphoric acid, and phosphorus acid esters having chlorine or bromine bonded directly to phosphorus and the formula:

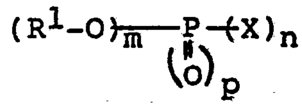

or

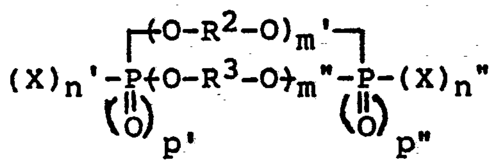

wherein

m is 1 to 3,

m' and m" are zero or 1 and are the same or different,

n is 1 to 4,

n' and n" are independently zero to 4 and are the same or different, with the proviso that n' and n" are both not zero,

p, p', and p" are zero or 1 and are the same or different,

$m+n+2p = 3$ or 5,

$m'+m"+n'+2p' = 3$ or 5,

$m'+m"+n"+2p" = 3$ or 5,

$R^1$ is a monovalent olefinic organic radical which can be straight chain, branched, or cyclic, can contain skeletal hetero atoms, and can be unsubstituted or substituted with non-interfering moieties,

$R^2$ and $R^3$ are divalent olefinic organic radicals

which can be straight chain, branched, or cyclic, can contain skeletal hetero atoms, can be unsubstituted or substituted with non-interferring moieties, and are the same or different, and

X is Cl, Br, or $R^4$, where $R^4$ is an aliphatic or oxyaliphatic radical having 1 to 12 carbon atoms, and each X is the same as or different from other X, with the proviso that at least one X is Cl or Br.

9. A method for repairing, veneering, or fastening dental appliances to hard dental tissue, without the necessity for acid etching said tissue, comprising the steps of:

(a) applying to said tissue a polymerizable composition comprising a free-radically polymerizable liquid monomer and a sulfur compound, and

(b) causing said composition to harden on said tissue,

characterized in that said polymerizable composition is a composition according to Claim 1.

10. A method according to Claim 9, further characterized in that said polymerizable liquid monomer comprises an organic ester of one or more acids of phosphorus having chlorine or bromine bonded directly to phosphorus, the organic radical of said ester containing at least one polymerizable functional group, and said salt of sulfurous acid comprises a bisulfite salt.